# EUROPEAN PATENT APPLICATION

(11) **EP 2 581 360 A1**
(43) Date of publication of application: **17.04.2013**
(21) Application number: 11795695.3
(22) Date of filing: 13.06.2011
(51) Int. Cl.: C07C 17/354, B01J 23/44, B01J 23/50, B01J 27/10, B01J 27/13, B01J 27/32, B01J 38/10, C07C 17/358, C07C 21/18, C07B 61/00

(54) **METHOD FOR PRODUCING FLUORINE COMPOUND**

(30) Priority: 14.06.2010 JP 2010134969
(71) Applicant: National Institute of Advanced Industrial Science and Technology, Tokyo 100-8921 (JP)
(72) Inventor: QUAN Heng-dao, Tsukuba-shi Ibaraki 305-8565 (JP); TAMURA Masanori, Tsukuba-shi Ibaraki 305-8565 (JP); MIZUKADO Junji, Tsukuba-shi Ibaraki 305-8565 (JP); SEKIYA Akira, Tsukuba-shi Ibaraki 305-8565 (JP)
(74) Representative: Wimmer, Hubert
(86) International application number: PCT/JP2011/063506
(87) International publication number: WO 2011/158790

(57) **Abstract**

An object is to provide a method for producing 1,1,1,4,4,4-hexafluoro-2-butene with high efficiency, which is suitable for a flow reaction, and cis-1,1,1,4,4,4-hexafluoro-2-butene can be efficiently obtained when isomerization of hexafluoro-1,3-butadiene is conducted using a catalyst and the resultant mixture is successively subjected to catalytic hydrogenation to produce cis-1,1,1,4,4,4-hexafluoro-2-butene, wherein the whole process is performed by a flow catalytic reaction.

## Description

### Technical Field

The present invention relates to a method for producing cis-1,1,1,4,4,4-hexafluoro-2-butene, and more particularly to a method for producing cis-1,1,1,4,4,4-hexafluoro-2-butene from hexafluoro-1,3-butadiene through hexafluoro-2-butyne.

### Background Art

Generally, fluorocompounds are widely used in commercial applications, such as polymer materials, refrigerants, detergents, pharmaceutical preparations, and agricultural chemicals. In the invention, unsaturated fluorocompounds are to be produced, and particularly, cis-1,1,1,4,4,4-hexafluoro-2-butene is produced, and these fluorocompounds are promising in the above-mentioned applications.

Properties of the unsaturated fluorocompounds, such as flammability, toxicity, and stability, vary greatly depending on their structures. Cis-1,1,1,4,4,4-hexafluoro-2-butene (boiling point: about 32°C) is totally different in boiling point from trans-1,1,1,4,4,4-hexafluoro-2-butene (boiling point: about 9°C) which is a geometrical isomer, and, for utilizing the properties of cis-1,1,1,4,4,4-hexafluoro-2-butene, a method for efficiently producing cis-1,1,1,4,4,4-hexafluoro-2-butene with high selectivity is needed.

As a method for producing 1,1,1,4,4,4-hexafluoro-2-butene, a reaction of 1,1,1,4,4,4-hexafluoro-2-iodobutane with a base, and the like have been known (e.g., non-patent document 1). However, only a trans compound can be nearly always obtained by the above method. Therefore, as a method for producing cis-1,1,1,4,4,4-hexafluoro-2-butene, a method in which 1,1,1,4,4,4-hexafluoro-2-butyne is subjected to catalytic hydrogenation is employed.

On the other hand, Henne et. al. have reported that hexafluoro-2-butyne is reduced with hydrogen under 100 atm. at room temperature using a Raney nickel catalyst to obtain cis-1,1,1,4,4,4-hexafluoro-2-butene, and that the yield of cis-1,1,1,4,4,4-hexafluoro-2-butene is, however, as low as 34% of the charge raw material and 1,1,1,4,4,4-hexafluorobutane, which is a side product caused due to excessive reduction, is formed (21% of the charge raw material) (non-patent document 2).
R. N. Hazeldine has reported that, by reducing hexafluoro-2-butyne with hydrogen under 15 atm. at 60°C using Raney nickel, cis-hexafluoro-2-butene can be obtained in a yield of 91% (non-patent document 1).
However, Raney nickel has properties such that it ignites in air, and therefore the use of this catalyst has a problem about the mass production with safety.

For solving the above problem, a method in which hexafluoro-2-butyne is hydrogenated using a Lindlar catalyst to obtain cis-1,1,1,4,4,4-hexafluoro-2-butene has been disclosed (patent document 1). The Lindlar catalyst used in this method is obtained by poisoning a catalyst having palladium supported on a calcium carbonate carrier with a lead compound, and thus the catalyst has a problem in that toxic lead must be used.

Further, a method in which hexafluoro-2-butyne is hydrogenated using a palladium catalyst in the presence of a non-aromatic amine catalyst modification agent to produce cis-1,1,1,4,4,4-hexafluoro-2-butene has been disclosed (patent document 2). In this report, an example in which toxic lead was used as a non-aromatic amine catalyst modification agent is shown in the working Examples. Further, this report shows that when hexafluoro-2-butyne is hydrogenated in a batchwise manner using a catalyst obtained by treating a catalyst having palladium supported on carbon with quinoline, or a catalyst having palladium supported on barium sulfate, excessively reduced 1,1,1,4,4,4-hexafluorobutane is mainly formed.

On the other hand, as a method for producing 1,1,1,4,4,4-hexafluoro-2-butyne which is used as a raw material for cis-1,1,1,4,4,4-hexafluoro-2-butene, there is an isomerization reaction of hexafluorobutadiene.
V. A. Petrov et. al. have obtained hexafluoro-2-butyne by subjecting hexafluorobutadiene to reaction in a batchwise manner using aluminum chlorofluoride (ACF) as a catalyst at 25°C for 2 hours (non-patent document 3). Aluminum chlorofluoride, which is obtained by a reaction of trichlorofluoromethane (CFC-11) with aluminum chloride, is in a powdery form and hence is not suitable for a flow reaction.

### Related Art References

### Patent Documents

Patent document 1: International Patent Application Publication No. 2009/142642
Patent document 2: International Patent Application Publication No. 2010/014548

### Non-patent Documents

Non-patent document 1: R. N. Hazeldine, J. Chem. Soc. 1952, pp. 2,504
Non-patent document 2: A. L. Heene et. al, J. Am. Chem. Soc., 71, 298 (1949)
Non-patent document 3: V. A. Petro, C. G. Krespan, B. E. smart, Journal Fluorine Chemistry, 77 (1996) 139-142

### Summary of Invention

### Problems that the Invention is to Solve

The present invention has been made for solving the above-mentioned problems accompanying the conventional techniques, and an object of the invention is to provide a method for producing 1,1,1,4,4,4-hexafluoro-2-butene with high efficiency, which is suitable for a flow reaction.

### Means for Solving the Problems

The present inventor has made extensive and intensive studies with a view toward achieving the above-mentioned object. As a result, it has been found that cis-1,1,1,4,4,4-hexafluoro-2-butene can be efficiently obtained when isomerization of hexafluoro-1,3-butadiene is conducted using a catalyst and the resultant mixture is successively subjected to catalytic hydrogenation to produce cis-1,1,1,4,4,4-hexafluoro-2-butene, wherein the whole process is performed by a flow catalytic reaction. Further, studies have been made on the catalyst used in each catalytic reaction, and, as a result, a catalyst suitable for a flow reaction has been found.

The present invention has been completed, based on the above finding, and provides the following invention.
[1] A method for producing cis-1,1,1,4,4,4-hexafluoro-2-butene from hexafluoro-1,3-butadiene, wherein cis-1,1,1,4,4,4-hexafluoro-2-butene is produced through hexafluoro-2-butyne by a flow catalytic reaction.
[2] The method according to item [1] above, which has the first step of conducting isomerization of hexafluoro-1,3-butadiene by a catalytic reaction to obtain hexafluoro-2-butyne.
[3] The method according to item [2] above, wherein the catalyst used in the first step is halogenated alumina.
[4] The method according to item [3] above, wherein the catalyst is obtained by reacting chlorofluorocarbon (CFCs), hydrochlorofluorocarbon (HCFCs), or hydrofluorocarbon (HFCs) with alumina at 20 to 600°C.
[5] The method according to any one of items [1] to [4] above, wherein the reaction temperature in the first step is 20 to 400°C.
[6] The method according to item [1] above, which has the second step of obtaining cis-1,1,1,4,4,4-hexafluoro-2-butene from hexafluoro-2-butyne by a catalytic hydrogenation reaction.
[7] The method according to item [6] above, wherein the catalyst used in the second step comprises at least one metal selected from palladium, copper, silver, and bismuth and a carrier having the metal supported thereon.
[8] The method according to item [7] above, wherein the carrier is aluminum fluoride, alumina, or activated carbon.
[9] The method according to item [6] above, wherein the catalyst comprises a mixture of palladium and bismuth and aluminum fluoride having the mixture supported thereon.
[10] The method according to item [6] above, wherein the catalyst is pretreated with an aromatic amine.
[11] The method according to item [10] above, wherein the aromatic amine is quinoline.
[12] The method according to any one of items [1] to [11] above, wherein the first step and the second step are performed in a single continuous step.
[13] The method according to item [12] above, wherein the isomerization of hexafluoro-1,3-butadiene to hexafluoro-2-butyne is conducted and the resultant composition is successively subjected to catalytic hydrogenation without purifying the composition.
[14] A method for producing hexafluoro-2-butyne from hexafluoro-1,3-butadiene, wherein isomerization of hexafluoro-1,3-butadiene is conducted by a flow catalytic reaction.
[15] The method according to item [14] above, wherein the catalyst is halogenated alumina.
[16] The method according to item [15] above, wherein the catalyst is obtained by reacting chlorofluorocarbon (CFCs), hydrochlorofluorocarbon (HCFCs), or hydrofluorocarbon (HFCs) with alumina at 20 to 600°C.
[17] The method according to item [16] above, wherein the temperature for the isomerization reaction of hexafluoro-1,3-butadiene to hexafluoro-2-butyne is 20 to 400°C.
[18] A method for producing cis-1,1,1,4,4,4-hexafluoro-2-butene by subjecting hexafluoro-2-butyne to catalytic hydrogenation reaction, wherein the reaction is conducted by a flow reaction, and wherein the catalyst for the hydrogenation comprises at least one metal selected from palladium, copper, silver, and bismuth and a carrier having the metal supported thereon.
[19] The method according to item [18] above, wherein the catalyst carrier is porous aluminum fluoride, alumina, or activated carbon.
[20] The method according to item [19] above, wherein the catalyst comprises a mixture of palladium and bismuth and aluminum fluoride having the mixture supported thereon.
[21] The method according to item [18] above, wherein the catalyst is pretreated with an aromatic amine.
[22] The method according to item [21] above, wherein the aromatic amine is quinoline.

### Advantage of the Invention

By the invention, the problems accompanying the conventional techniques are solved, and cis-1,1,1,4,4,4-hexafluoro-2-butene can be efficiently produced by a flow reaction method which is suitable for the production on a commercial scale.

### Mode for Carrying Out the Invention

The invention is a method for producing cis-1,1,1,4,4,4-hexafluoro-2-butene by conducting isomerization of hexafluoro-1,3-butadiene using a catalyst to form hexafluoro-2-butyne, and successively subjecting the resultant hexafluoro-2-butyne to catalytic hydrogenation, wherein the whole process is performed by a flow catalytic reaction.

In the invention, the production of cis-1,1,1,4,4,4-hexafluoro-2-butene may be performed in two steps, i.e., a step (first step) of conducting isomerization of hexafluoro-1,3-butadiene to obtain hexafluoro-2-butyne and a step (second step) of subjecting hexafluoro-2-butyne to catalytic hydrogenation to obtain cis-1,1,1,4,4,4-hexafluoro-2-butene, or in a single continuous step of conducting isomerization of hexafluoro-1,3-butadiene and successively subjecting hexafluoro-2-butyne as an intermediate to catalytic hydrogenation without purifying the intermediate. The production is preferably performed in a single step without purifying the hexafluoro-2-butyne as an intermediate.

The above-mentioned step of conducting isomerization of hexafluoro-1,3-butadiene (HFBD) to produce hexafluoro-2-butyne is performed by a flow reaction.
This isomerization reaction is conducted in the presence of a catalyst, and, as a catalyst, preferred is a halogenated alumina, and examples include fluorinated alumina, chlorinated alumina, brominated alumina, iodinated alumina, chlorinated fluorinated alumina, brominated fluorinated alumina, and chlorinated brominated alumina, and preferred is chlorinated fluorinated alumina.
With respect to the method for producing the halogenated alumina, there is no particular limitation, but it is preferred that the halogenated alumina is produced by a reaction of alumina with chlorofluorocarbon (CFCs), hydrochlorofluorocarbon (HCFCs), or hydrofluorocarbon (HFCs). The temperature for the production is generally 20 to 600°C, preferably 300 to 400°C.
The temperature for the reaction of hexafluoro-1,3-butadiene to form hexafluoro-2-butyne is generally 20 to 400°C, preferably 50 to 200°C.

The step of producing cis-1,1,1,4,4,4-hexafluoro-2-butene from hexafluoro-2-butyne by a catalytic hydrogenation reaction is also performed by a flow reaction.
The catalyst used in the catalytic hydrogenation reaction comprises at least one metal selected from palladium, copper, silver, and bismuth and a carrier, preferably alumina, porous aluminum fluoride, or activated carbon, having the metal supported thereon. The catalyst more preferably comprises a mixture of palladium and bismuth and porous aluminum fluoride having the mixture supported thereon. Further, the catalyst may be pretreated with an aromatic amine, preferably pretreated with quinoline.
The temperature for the catalytic hydrogenation reaction is generally 20 to 350°C, preferably 150 to 250°C.

Hexafluoro-2-butene can also be obtained from hexafluoro-1,3-butadiene (HFBD) through hexafluoro-2-butyne (HFB) in a single continuous step using the above-mentioned catalytic isomerization reaction and catalytic hydrogenation reaction in combination.

### Examples

Hereinbelow, the present invention will be described in more detail with reference to the following Examples, which should not be construed as limiting the scope of the invention.

### [Example 1]

### (Production of catalyst A)

18 ml of alumina was placed in a reactor tube having a diameter of 14 mm and a length of 300 mm. The reactor tube was heated to 400°C in a nitrogen gas stream at 100 ml/min. Then, dichlorodifluoromethane (CFC-12) was passed through the reactor tube at 100 ml/min at 400°C for 3 hours to obtain a catalyst A. The catalyst A had a surface area of 72.56 m2/g.

### (Production of hexafluoro-2-butyne)

The catalyst A was placed in the above-mentioned reactor tube. Hexafluoro-1,3-butadiene was measured by means of a mass flowmeter and passed through the reactor tube. A reaction was conducted at 20 to 150°C, and the resultant product was passed through a dryer and an online GC, collecting a final product in a trap at -100°C.
The experimental results are shown in Table 1 below.

**[Table 1]**

| Entry | Reaction temperature °C | Contact time s | Flow rate ml/min | Yield of hexafluoro-2-butyne % |
|---|---|---|---|---|
| 1 | 20 | 96 | 10 | 99.5 |
| 2 | 100 | 96 | 10 | 99.5 |
| 3 | 100 | 32 | 30 | 95.3 |
| 4 | 150 | 32 | 30 | 99.6 |

### [Example 2]

### (Production of catalyst B)

Porous aluminum fluoride (PAF) was impregnated with a satisfactory amount of a palladium chloride solution overnight. The amount of the metal chloride in the solution was adjusted so that the amount of the finally supported metal became about 3% by weight. After the impregnation, the carrier having palladium supported thereon was heated at 200°C for 6 hours and further at 300°C for 6 hours, and then reduced in a hydrogen gas stream at a flow rate of 20 ml/min at 200°C for 6 hours, at 300°C for 6 hours, and further at 350°C for 5 hours to obtain a catalyst B.

### (Production of cis-1,1,1,4,4,4-hexafluoro-2-butene)

The catalyst B was placed in the above-mentioned reactor tube. Hexafluoro-2-butyne was measured by means of a mass flowmeter and passed through the reactor tube. A reaction was conducted at 20 to 250°C, and the resultant product was passed through a dryer and an online GC, collecting a final product in a trap at -100°C.
The experimental results are shown in Table 2 below.
In Tables 2 to 6 below, numerals 1 to 4 shown in the columns below "GC Area %" indicate, respectively, compounds 1 to 4 shown in the following formulae.

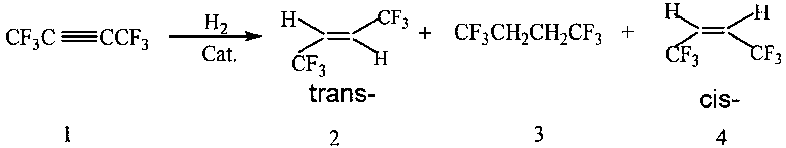

**[Table 2]**

| No. | Reaction temperature °C | GC Area % | | | |
|---|---|---|---|---|---|
| | | 1 | 2 | 3 | 4 |
| 1 | 20 | 29.7 | 5.8 | 2.6 | 61.9 |
| 2 | 50 | 26.8 | 5.4 | 2.0 | 65.8 |
| 3 | 100 | 28.3 | 4.8 | 1.4 | 65.5 |
| 4 | 150 | 35.5 | 4.8 | 1.3 | 58.4 |
| 5 | 200 | 35.5 | 4.2 | 1.4 | 58,9 |
| 6 | 250 | 41.4 | 10.3 | 1.7 | 46.6 |

| | | | | | |
|---|---|---|---|---|---|
| Note: Reaction time: 10.5 S; Hexafluoro-2-butyne flow rate: 10 ml/min Hydrogen flow rate: 10 ml/min; Nitrogen flow rate: 10 ml/min. | | | | | |

### [Example 3]

### (Production of catalyst C)

Porous aluminum fluoride (PAF) was impregnated with a satisfactory amount of a solution of palladium chloride and bismuth chloride overnight. The amounts of the metal chlorides in the solution were adjusted so that the amount of the supported palladium became about 2% by weight and the amount of the supported bismuth became about 0.1 %. The carrier having palladium supported thereon was heated at 200°C for 6 hours and further at 300°C for 6 hours, and then reduced in a hydrogen gas stream at a flow rate of 20 ml/min at 200°C for 6 hours, at 300°C for 6 hours, and further at 350°C for 5 hours to obtain a catalyst C.

### (Production of cis-1,1,1,4,4,4-hexafluoro-2-butene)

The catalyst C was placed in the above-mentioned reactor tube. Hexafluoro-2-butyne was measured by means of a mass flowmeter and passed through the reactor tube. A reaction was conducted at 20 to 250°C, and the resultant product was passed through a dryer and an online GC, collecting a final product in a trap at -100°C.
The experimental results are shown in Table 3 below.

**[Table 3]**

| No. | Reaction temperature °C | GC Area % | | | |
|---|---|---|---|---|---|
| | | 1 | 2 | 3 | 4 |
| 1 | 20 | 9.4 | 3.3 | 8.5 | 78.8 |
| 2 | 50 | 4.9 | 4.3 | 7.9 | 82.9 |
| 3 | 100 | 4.4 | 4.8 | 4.4 | 83.6 |
| 4 | 150 | 3.6 | 4.7 | 5.6 | 86.1 |
| 5 | 200 | 2.5 | 4.2 | 4.1 | 89.2 |
| 6 | 250 | 1.3 | 7.2 | 2.5 | 89.0 |

| | | | | | |
|---|---|---|---|---|---|
| Note: Reaction time: 6.7 S; Hexafluoro-2-butyne flow rate: 6 ml/min; Hydrogen flow rate: 6 ml/min; Nitrogen flow rate: 6 ml/min. | | | | | |

### [Example 4]

### (Production of catalyst D)

Activated carbon was impregnated with a satisfactory amount of a solution of palladium chloride and silver nitrate overnight. The amounts of the metals in the solution were adjusted so that the amount of the supported palladium became about 4.5% by weight and the amount of the supported silver became about 0.5%. The carrier having palladium supported thereon was heated at 200°C for 6 hours and further at 300°C for 6 hours, and then reduced in a hydrogen gas stream at a flow rate of 20 ml/min at 200°C for 6 hours, at 300°C for 6 hours, and further at 350°C for 5 hours to obtain a catalyst D.

### (Production of cis-1,1,1,4,4,4-hexafluoro-2-butene)

The catalyst D was placed in the above-mentioned reactor tube. Hexafluoro-2-butyne was measured by means of a mass flowmeter and passed through the reactor tube. A reaction was conducted at 20 to 250°C, and the resultant product was passed through a dryer and an online 0GC, collecting a final product in a trap at -100°C.
The experimental results are shown in Table 4 below.

**[Table 4]**

| No. | Reaction temperature °C | GC Area % | | | |
|---|---|---|---|---|---|
| | | 1 | 2 | 3 | 4 |
| 1 | RT | 21.7 | 7.9 | 30.1 | 40.3 |
| 2 | 50 | 19.7 | 8.5 | 32.0 | 39.9 |
| 3 | 100 | 19.3 | 12.0 | 25.7 | 42.9 |
| 4 | 150 | 17.4 | 13.3 | 23.5 | 45.8 |
| 5 | 200 | 15.6 | 13.6 | 22.6 | 48.6 |
| 6 | 250 | 13.4 | 16.0 | 22.0 | 48.6 |

| | | | | | |
|---|---|---|---|---|---|
| Note: Reaction time: 5.7 S; Hexafluoro-2-butyne flow rate: 6 ml/min; Hydrogen flow rate: 6 ml/min; Nitrogen flow rate: 6 ml/min. | | | | | |

### [Example 5]

### (Production of catalyst E)

Alumina was impregnated with a satisfactory amount of palladium chloride overnight. The amount of the metal in the solution was adjusted so that the amount of the supported palladium became about 1 % by weight. The carrier having palladium supported thereon was heated at 200°C for 6 hours and further at 300°C for 6 hours, and then reduced in a hydrogen gas stream at a flow rate of 20 ml/min at 200°C for 6 hours, at 300°C for 6 hours, and further at 350°C for 5 hours to obtain a catalyst E.

### (Production of cis-1,1,1,4,4,4-hexafluoro-2-butene)

The catalyst E was placed in the above-mentioned reactor tube. Hexafluoro-2-butyne was measured by means of a mass flowmeter and passed through the reactor tube. A reaction was conducted at 20 to 250°C, and the resultant product was passed through a dryer and an online GC, collecting a final product in a trap at -100°C.
The experimental results are shown in Table 5 below.

**[Table 5]**

| No. | Reaction temperature °C | GC Area % | | | |
|---|---|---|---|---|---|
| | | 1 | 2 | 3 | 4 |
| 1 | 20 | 18.6 | 12.4 | 24.3 | 44.7 |
| 2 | 50 | 16.0 | 13.9 | 21.6 | 48.4 |
| 3 | 100 | 13.6 | 14.4 | 18.8 | 53.2 |
| 4 | 150 | 10.9 | 14.4 | 15.4 | 59.3 |
| 5 | 200 | 8.0 | 14.4 | 11.5 | 66.0 |
| 6 | 250 | 5.4 | 14.0 | 8.4 | 72.2 |

| | | | | | |
|---|---|---|---|---|---|
| Note: Reaction time: 6.7 S; Hexafluoro-2-butyne flow rate: 6 ml/min; Hydrogen flow rate: 6 ml/min; Nitrogen flow rate: 6 ml/min. | | | | | |

### [Example 6]

### (Production of catalyst F)

Alumina was impregnated with a satisfactory amount of palladium chloride overnight. The amount of the metal in the solution was adjusted so that the amount of the supported palladium became about 1 % by weight. The carrier having palladium supported thereon was heated at 200°C for 6 hours and further at 300°C for 6 hours, and then reduced in a hydrogen gas stream at a flow rate of 20 ml/min at 200°C for 6 hours, at 300°C for 6 hours, and further at 350°C for 5 hours. Finally, the resultant catalyst was treated with a flow of quinoline at 250°C for 2 hours to obtain a catalyst F.

### (Production of cis-1,1,1,4,4,4-hexafluoro-2-butene)

The catalyst F was placed in the above-mentioned reactor tube. Hexafluoro-2-butyne was measured by means of a mass flowmeter and passed through the reactor tube. A reaction was conducted at 20 to 250°C, and the resultant product was passed through a dryer and an online GC, collecting a final product in a trap at -100°C.
The experimental results are shown in Table 6 below.

**[Table 6]**

| No. | Reaction temperature °C | GC Area % | | | |
|---|---|---|---|---|---|
| | | 1 | 2 | 3 | 4 |
| 1 | RT | 100 | 0 | 0 | 0 |
| 2 | 100 | 47.1 | 0.3 | 2.5 | 49.3 |
| 3 | 150 | 18.4 | 1.0 | 4.5 | 75.2 |
| 4 | 200 | 13.9 | 1.4 | 4.6 | 79.1 |
| 5 | 250 | 8.6 | 2.5 | 5.4 | 83.6 |

| | | | | | |
|---|---|---|---|---|---|
| Note: Reaction time: 6.7 S; Hexafluoro-2-butyne flow rate: 6 ml/min; Hydrogen flow rate: 6 ml/min; Nitrogen flow rate: 6 ml/min. | | | | | |

### [Example 7]

### (Production of cis-1,1,1,4,4,4-hexafluoro-2-butene from hexafluorobutadiene)

18 ml of a catalyst A was placed in a first reactor tube having a diameter of 14 mm and a length of 300 mm, and a catalyst C was placed in a second reactor tube. Hexafluoro-1,3-butadiene (15 ml/min) was measured by means of a mass flowmeter and passed through the first reactor tube to conduct a reaction at 100°C. The resultant product stream was successively passed through the second reactor tube to conduct a reaction at 200°C. The resultant product was passed through a dryer and an online GC, collecting a final product in a trap at -100°C.

As a result, a mixture having the formulation shown below (each amount indicated by GC area %) was obtained.

| | |
|---|---|
| Hexafluoro-1,3-butadiene | 2.5% |
| Trans-1,1,1,4,4,4-hexafluoro-2-butene | 4.2% |
| 1,1,1,4,4,4-Hexafluorobutane | 4.1% |
| Cis-1,1,1,4,4,4-hexafluoro-2-butene | 89.2% |

## Claims

1. A method for producing cis-1,1,1,4,4,4-hexafluoro-2-butene from hexafluoro-1,3-butadiene, wherein cis-1,1,1,4,4,4-hexafluoro-2-butene is produced through hexafluoro-2-butyne by a flow catalytic reaction.

2. The method according to claim 1, which has the first step of conducting isomerization of hexafluoro-1,3-butadiene by a catalytic reaction to obtain hexafluoro-2-butyne.

3. The method according to claim 2, wherein the catalyst used in the first step is halogenated alumina.

4. The method according to claim 3, wherein the catalyst is obtained by reacting chlorofluorocarbon (CFCs), hydrochlorofluorocarbon (HCFCs), or hydrofluorocarbon (HFCs) with alumina at 20 to 600°C.

5. The method according to any one of claims 1 to 4, wherein the reaction temperature in the first step is 20 to 400°C.

6. The method according to claim 1, which has the second step of obtaining cis-1,1,1,4,4,4-hexafluoro-2-butene from hexafluoro-2-butyne by a catalytic hydrogenation reaction.

7. The method according to claim 6, wherein the catalyst used in the second step comprises at least one metal selected from palladium, copper, silver, and bismuth and a carrier having the metal supported thereon.

8. The method according to claim 7, wherein the carrier is aluminum fluoride, alumina, or activated carbon.

9. The method according to claim 6, wherein the catalyst comprises a mixture of palladium and bismuth and aluminum fluoride having the mixture supported thereon.

10. The method according to claim 6, wherein the catalyst is pretreated with an aromatic amine.

11. The method according to claim 10, wherein the aromatic amine is quinoline.

12. The method according to any one of claims 1 to 11, wherein the first step and the second step are performed in a single continuous step.

13. The method according to claim 12, wherein the isomerization of hexafluoro-1,3-butadiene to hexafluoro-2-butyne is conducted and the resultant composition is successively subjected to catalytic hydrogenation without purifying the composition.

14. A method for producing hexafluoro-2-butyne from hexafluoro-1,3-butadiene, wherein isomerization of hexafluoro-1,3-butadiene is conducted by a flow catalytic reaction.

15. The method according to claim 14, wherein the catalyst is halogenated alumina.

16. The method according to claim 15, wherein the catalyst is obtained by reacting chlorofluorocarbon (CFCs), hydrochlorofluorocarbon (HCFCs), or hydrofluorocarbon (HFCs) with alumina at 20 to 600°C.

17. The method according to claim 16, wherein the temperature for the isomerization reaction of hexafluoro-1,3-butadiene to hexafluoro-2-butyne is 20 to 400°C.

18. A method for producing cis-1,1,1,4,4,4-hexafluoro-2-butene by subjecting hexafluoro-2-butyne to catalytic hydrogenation reaction, wherein the reaction is conducted by a flow reaction, and wherein the catalyst for the hydrogenation comprises at least one metal selected from palladium, copper, silver, and bismuth and a carrier having the metal supported thereon.

19. The method according to claim 18, wherein the carrier is porous aluminum fluoride, alumina, or activated carbon.

20. The method according to claim 19, wherein the catalyst comprises a mixture of palladium and bismuth and aluminum fluoride having the mixture supported thereon.

21. The method according to claim 18, wherein the catalyst is pretreated with an aromatic amine.

22. The method according to claim 21, wherein the aromatic amine is quinoline.
